# EUROPEAN PATENT APPLICATION

(11) **EP 4 014 990 A1**
(43) Date of publication of application: **22.06.2022**
(21) Application number: 20216195.6
(22) Date of filing: 21.12.2020
(51) Int. Cl.: A61K 39/275, C12N 15/86

(54) **MODIFIED PARAPOXVIRUS HAVING INCREASED IMMUNOGENICITY**

(71) Applicant: Eberhard Karls Universität Tübingen Medizinische Fakultät, 72074 Tübingen (DE)
(72) Inventor: Amann, Ralf, 72070 Tübingen (DE); Salomon, Ferdinand, 72072 Tübingen (DE)
(74) Representative: Witte, Weller & Partner Patentanwälte mbB

(57) **Abstract**

The present invention relates to a modified *Parapoxvirus,* preferably a *Parapoxvirus* vector, having an increased immunogenicity, a biological cell containing said modified *Parapoxvirus,* a pharmaceutical composition, preferably a vaccine, containing said modified *Parapoxvirus* and/or said cell, and a new use of said modified *Parapoxvirus.*

SEQUENCE LISTING

<110>  Eberhard Karls Universität Tübingen, Medizinische Fakultät

<120>  Parapoxvirus vector having increased immunogenicity

<130>  5402P615EP

<160>  8

<170>  PatentIn version 3.5

<210>  1
<211>  16
<212>  DNA
<213>  Artificial Sequence

<220>
<223>  P1 Promoter

<400>  1
aaaaattgaa aaatta                                                    16


<210>  2
<211>  16
<212>  DNA
<213>  Artificial Sequence

<220>
<223>  P2 Promoter

<400>  2
aaaaattgaa attcta                                                    16


<210>  3
<211>  16
<212>  DNA
<213>  Artificial Sequence

<220>
<223>  "Optimized Early" Promoter

<400>  3
aaaaattgaa aaayta                                                    16


<210>  4
<211>  15
<212>  DNA
<213>  Artificial Sequence

<220>
<223>  7.5 kDa Promoter

<400>  4
aaaagtagaa aatta                                                     15


<210>  5
<211>  16
<212>  DNA

<213>  Artificial Sequence

<220>
<223>  VEGF Promoter

<400>  5
caaaatgtaa attata                                                    16


<210>  6
<211>  2331
<212>  DNA
<213>  Artificial Sequence

<220>
<223>  D1701-V ORF111 - 112 - 113

<400>  6
atgtcgcgac ttcaaatact gacctcattt ggacaaatct acgcacccga cgaagctcgg      60

ctgcgcgaga tcgcgcgtga tttgggaata tgcaccataa aacgcgcatt cggcgacatg     120

ctgtacggct ttatagactt caacccggtg cccctgaccc aagtaaacat gctcatgccc     180

aactgctact tcgcggtcaa cggcaacctg cttccgtgca cggaggactt ccggctcaga     240

ctcccggcaa cggagatctc tgcggcctac ctgacgaaaa cgggacggac gatcctgtgc     300

ggcagagact tcaacatagt agcgccgtcg gggttcaagc cgtccatgcg gctgcgcaac     360

ctcagtcacg tgtctgcgct tgtagagatt ctagagcttt acgacgagtc cggagagtac     420

caattcgtgc tcggccccag cgcgcagtta atgctgcggc taatggagaa ggagaatgtc     480

tgtctgtttg gcagaggttg gtgcatagtg gacctgcgca agctggacgt ggccatataa     540

ttgctgctgc tatgtcgtgc ccgactctgt gcgacaaaga agcagcttac cagacgactc     600

ttcgttcctg ttctccaagc aagaacactg gagtgagttg ccatttccgt ctccaaccat     660

ataattagca tccttgtttt tatcctgtat tttatcagtt tttagctagt taaaacataa     720

atagtaaagc tataaagagg agttctggaa tcttgcaacg acaggatgaa ggcggtgttg     780

ttgctatcgc tactgggagc gttcaccaac gcagcgcctt tgttaagcaa ccagcgtctt     840

ggcagtgcag aagaagaaaa tttctgctcg acgcatcagg gcgaagtgca cgccaggttc     900

tggcttcaga tgcgcgtggg tgtacgacac agtccgcttt acactcccag caacatgtgc     960

atgatggaca tagaagactc cattacggac acagaagact ccacagaaaa agaatacacg    1020

tctacggcaa cgggtgatgc ggacggattg aacgtgtccg tggcactaat tggagaaggc    1080

gtgagcatac cgctaagtta cataggcctc ggattcaacc cgtcgcttac agatggctac    1140

ctgtacgtca acgtctcgtc acgggctcct tgggatcaac agactctgga cctatccgcg    1200

aacgacggct ggggtatcaa acaggttcta gaaaaagaga tactggccat ccagataggg    1260

tgcgacaacc aaaaatttcc cgaagaaccc acaactaccc aacccccctc acctgtcaca    1320

acaacgcttt cctcaacaac tctagatccg aatgacaaaa acacagacac tacgccgaca    1380

accaccggcg ccagtgtaga cggaaagcgc aatccagatg actttgactt ctcgctgatc 1440

gtggaccccc gatgcgtgac ctctgtaaac ctgcactttg agattaagga cgcgtgcatg 1500

gaccacaaag aatcctcgcc gttgtcgctg aagggggaat atggagacgg cgaactagta 1560

agaaaagaaa tcaaagacgt gggaaaggat cacaatatgt gcagtcttaa cctcagccct 1620

ggccattgag ctgtttttat tcggcaatat aataggtgat tattgaacat taaacaaaac 1680

ttatcccaca acgccgcaac aatggaagtg atggtgaccg tctctattat tgtcgccgta 1740

atatgcttaa ctggaacggc aatatacctc cttattgaac tcggcttagc cgccgagcgc 1800

gctaacaaac gagcgcgcgt aaagaaaaat atgcgcaaat tagccactca attgggaaat 1860

ggatctgtcg actccggaat aggcccgtgc acaatatcgc gcaccatgga ctttggacct 1920

agtcgctggg acagcgacag tgagggtgac ggagacagca tgtccacgac gtccactagc 1980

ggagggggga ctctcacccg agtgtgggtt gggagcgggt ccgggcccat gtacgaaaac 2040

ttttgcggga acggcaccca ccgccactcc cccaccaacg accctggata ccactcgcgg 2100

gagactctct gcagcggacc tccccgtcag gcgccggcgc tacctctcac tccgaagcct 2160

gacgaggtag aggtggacgt agggcctggt cccgacgata gatacaccgg cccgtacgag 2220

gaacctgatc ccattctcct gcaggaacct gagccggtgc agattgaggt aaccatcaac 2280

gggcccggtg gagaaggcga ggcggaggga gaatttttct acgacgagta g 2331

<210> 7
<211> 2057
<212> DNA
<213> Artificial Sequence

<220>
<223> pDel 112-2-AcGFP

<400> 7
acctcatttg gacaaatcta cgcacccgac gaagctcggc tgcgcgagat cgcgcgtgat 60

ttgggaatat gcaccataaa acgcgcattc ggcgacatgc tgtacggctt tatagacttc 120

aacccggtgc ccctgaccca agtaaacatg ctcatgccca actgctactt cgcggtcaac 180

ggcaacctgc ttccgtgcac ggaggacttc cggctcagac tcccggcaac ggagatctct 240

gcggcctacc tgacgaaaac gggacggacg atcctgtgcg gcagagactt caacatagta 300

gcgccgtcgg ggttcaagcc gtccatgcgg ctgcgcaacc tcagtcacgt gtctgcgctt 360

gtagagattc tagagcttta cgacgagtcc ggagagtacc aattcgtgct cggccccagc 420

gcgcagttaa tgctgcggct aatggagaag gagaatgtct gtctgtttgg cagaggttgg 480

tgcatagtgg acctgcgcaa gctggacgtg gccatataat tgctgctgct atgtcgtgat 540

atcaaaattg gaaattctaa tacattaaat tataggatcc ggtaccacta gtcttaagat 600

cgatttttta tcttgttttt atcctgtctt tttatcagtt ttttagctag ttaaaacata 660

aatagtaaag ctaaaaagag actatatcgg cggctggagt cttgcaacaa ccagctccgg 720

aaaaaattga aattctaact tgtgttctta taaatgatca attgagatct ttcgaaaccg 780

gtcgccacca tggtgagcaa gggcgccgag ctgttcaccg gcatcgtgcc catcctgatc 840

gagctgaatg gcgatgtgaa tggccacaag ttcagcgtga gcggcgaggg cgagggcgat 900

gccacctacg gcaagctgac cctgaagttc atctgcacca ccggcaagct gcctgtgccc 960

tggcccaccc tggtgaccac cctgagctac ggcgtgcagt gcttctcacg ctaccccgat 1020

cacatgaagc agcacgactt cttcaagagc gccatgcctg agggctacat ccaggagcgc 1080

accatcttct tcgaggatga cggcaactac aagtcgcgcg ccgaggtgaa gttcgagggc 1140

gataccctgg tgaatcgcat cgagctgacc ggcaccgatt tcaaggagga tggcaacatc 1200

ctgggcaata agatggagta caactacaac gcccacaatg tgtacatcat gaccgacaag 1260

gccaagaatg gcatcaaggt gaacttcaag atccgccaca acatcgagga tggcagcgtg 1320

cagctggccg accactacca gcagaatacc cccatcggcg atggccctgt gctgctgccc 1380

gataaccact acctgtccac ccagagcgcc ctgtccaagg accccaacga gaagcgcgat 1440

cacatgatct acttcggctt cgtgaccgcc gccgccatca cccacggcat ggatgagctg 1500

tacaagtgag cggccgctga tcaacgcgtt ttttatcctg cagggctagc cggccggcat 1560

gctttttatg cgatcgccaa tggaagtgat ggtgaccgtc tctattattg tcgccgtaat 1620

atgcttaact ggaacggcaa tatacctcct tattgaactc ggcttagccg ccgagcgcgc 1680

taacaaacga gcgcgcgtaa agaaaaatat gcgcaaatta gccactcaat tgggaaatgg 1740

atctgtagac tccggaatag gcccgtgcac aatatcgcgc accatggact ttggacctag 1800

tcgctgggac agcgacagtg agggtgacgg agacagcatg tccacgacgt ccactagcgg 1860

aggggggact ctcacccgag tgtgggttgg gagcgggtcc gggcccatgt acgaaaactt 1920

ttgcgggaac ggcacccacc gccactcccc caccaacgac cctggatacc actcgcggga 1980

gactctctgc agcggacctc cccgtcaggc gccggcgcta cctctcactc cgaagcctga 2040

cgaggtagag gtggacg 2057

<210> 8
<211> 1132
<212> DNA
<213> Artificial Sequence

<220>
<223> pDel 112

<400> 8
acctcatttg gacaaatcta cgcacccgac gaagctcggc tgcgcgagat cgcgcgtgat 60

ttgggaatat gcaccataaa acgcgcattc ggcgacatgc tgtacggctt tatagacttc 120

aacccggtgc ccctgaccca agtaaacatg ctcatgccca actgctactt cgcggtcaac 180

ggcaacctgc ttccgtgcac ggaggacttc cggctcagac tcccggcaac ggagatctct 240

gcggcctacc tgacgaaaac gggacggacg atcctgtgcg gcagagactt caacatagta 300

gcgccgtcgg ggttcaagcc gtccatgcgg ctgcgcaacc tcagtcacgt gtctgcgctt 360

gtagagattc tagagcttta cgacgagtcc ggagagtacc aattcgtgct cggccccagc 420

gcgcagttaa tgctgcggct aatggagaag gagaatgtct gtctgtttgg cagaggttgg 480

tgcatagtgg acctgcgcaa gctggacgtg gccatataat tgctgctgct atgtcgtgat 540

atcaaaattg gaaattctaa tacattaaat tataggatcc ggtaccacta gtatcgatac 600

gcgtttttta tcctgcaggg ctagccggcc ggcatgcttt ttatgcgatc gccaatggaa 660

gtgatggtga ccgtctctat tattgtcgcc gtaatatgct taactggaac ggcaatatac 720

ctccttattg aactcggctt agccgccgag cgcgctaaca aacgagcgcg cgtaaagaaa 780

aatatgcgca aattagccac tcaattggga aatggatctg tagactccgg aataggcccg 840

tgcacaatat cgcgcaccat ggactttgga cctagtcgct gggacagcga cagtgagggt 900

gacggagaca gcatgtccac gacgtccact agcggagggg ggactctcac ccgagtgtgg 960

gttgggagcg ggtccggccc catgtacgaa aacttttgcg ggaacggcac ccaccgccac 1020

tcccccacca acgaccctgg ataccactcg cgggagactc tctgcagcgg acctccccgt 1080

caggcgccgg cgctacctct cactccgaag cctgacgagg tagaggtgga cg 1132

## Description

The present invention relates to a modified *Parapoxvirus,* preferably a *Parapoxvirus* vector, having an increased immunogenicity, a biological cell containing said modified *Parapoxvirus,* a pharmaceutical composition, preferably a vaccine, containing said modified *Parapoxvirus* vector and/or said cell, and a new use of said modified *Parapoxvirus.*

Modified viruses, e.g. viral vectors, have multiple applications in biosciences, medicine and process engineering. For instance, viral vector-based vaccines are promising to elicit cellular and humoral immune responses to any kind of antigen peptides. Within the genus *Parapoxvirus* of the *Poxviridae* the *Parapoxvirus ovis (Orf* virus; ORFV) strain D1701-V comprises various properties particularly favorable for the development of a vector platform technology and was shown to facilitate various vaccination approaches.

One problem which can be found in the art of modified viruses or viral vectors, however, is low immunogenicity. The low immunogenicity reduces the effectivity of viral vector-based vaccines. Current strategies to address this problem use the implementation of immunomodulatory elements or the formulation of the viral vectors in conjunction with pharmacological or immunological agents which improve the immune response of a vaccine. However, these approaches have not been fully successful. Some viral vectors are of limited packaging capacity and the expression of immunomodulatory elements quite often render the viruses inoperable. Furthermore, adjuvants are frequently toxic to the vaccinated organism that is why viral vector-based vaccination is often associated with side effects.

Against this background there is a need in the art to provide new modified viruses, in particular viral vectors, which can be used for the effective production of viral vector-based vaccines and other applications, where the problems known in the art are reduced or even avoided.

Therefore, it is an object underlying the invention to provide such a modified virus or viral vector, respectively, which is characterized by an increased immunogenicity without requiring the inclusion of immunomodulatory elements or the formulation of the vector with adjuvants.

The invention satisfies these and other needs by providing a modified *Parapoxvirus,* preferably a *Parapoxvirus* vector, comprising at least one functional mutation in the viral open reading frame (ORF) encoding the 'chemokine binding protein' (CBP), wherein said vector comprises increased immunogenicity in comparison with the same vector without said functional mutation.

According to the invention, *'Parapoxvirus'* is a genus of viruses in the family of *Poxviridae* and the subfamily of *Chordopoxvirinae.* Like all members of the family *Poxviridae* they are oval, relatively large, double-stranded DNA viruses. Parapoxviruses have a unique spiral coat that distinguishes them from other poxviruses. Parapoxviruses infect vertebrates, including a wide selection of mammals, and humans. According to the invention all kinds of Parapoxviruses are suitable, however, *Orf* viruses are of preference.

According to the invention, 'modified' *Parapoxvirus* refers to a *Parapoxvirus*-derived virus which has been technically modified over the wild type counterpart.

According to the invention *a'Parapoxvirus* vector' refers to a vector or plasmid based on or consisting of the *Parapoxvirus* genome, which is configured for the transfection of biological cells, preferably of mammalian and human cells, and further preferably also for the transport and/or the expression of a transgene or foreign gene in(to) biological cells.

According to the invention 'chemokine binding protein' (CBP) refers to a group of proteins encoded and expressed by pathogens, such as *parapoxviruses.* CBPs are unrelated to host receptors and bind chemokines with high affinity and block their activity. CBPs are part of a counter strategy to escape the host's antiviral immune response. To attenuate inflammation of infected cells, the *Orthopoxvirus* and *Leporipoxvirus* genera produce the poxvirus type II CC-chemokine binding proteins (CBP-II) that share a 17% sequence identity to the CBP encoded by *Orf* virus (ORF112). The latter CBP has been shown to be functionally similar to the poxviral CBP-II in its ability to bind lymphotactin or many human inflammatory and constitutive CC-chemokines such as CCL19 or CCL21 with high affinity and thus, to play a critical role in virulence and pathogenesis. An overview on CBPs is given in Gonzales-Motos et al. (2016), Chemokine binding proteins: An immunomodulatory strategy going viral, FASEB J. 18(3), pp. 571-573. The CBP of ORFV strain NZ2 is disclosed in Fleming et al. (2017), Deletion of the chemokine binding protein gene from the Parapoxvirus Orf virus reduces virulence and pathogenesis in sheep, Front. Microbiol. 8, p. 46.

According to the invention, a 'functional mutation' refers to a genetic alteration of a gene and the encoded protein which, in comparison to the non-mutated wild type, results in a change of activity and/or function. The functional mutation includes, without being limited thereto, knockout, point, deletion, frame shift, and substitution mutations etc., which all result in the alteration of CBP function, whether a dysfunctional CBP will be the outcome or the expression of CBP is suppressed, reduced or avoided, respectively.

According to the invention, 'immunogenicity' is the ability of the *Parapoxvirus* vector to provoke an immune response in the body of an animal of human being, i.e. the ability to induce humoral and/or cell-mediated immune responses. The immunogenicity can be measured by various methods well known to the skilled person. An overview on such methods can be found in Madhwa et al. (2015), Immunogenicity assessment of biotherapeutic products: An overview of assays and their utility, Biologicals Vol. 43, Is. 5, pp. 298-306.

The inventors were able to realize that a *Parapoxvirus* vector having an functionally mutated CBP, preferably inactivated CBP, is characterized by a particular solid and elevated immunogenicity in comparison with a non-mutated counterpart vector or wild type virus, respectively. Increased immunogenicity was demonstrated by the vector's ability to activate peripheral blood mononuclear cells, or to induce antigen-specific immune responses *in vitro.* At the same time, the inventors found that the modified *Parapoxvirus* or *Parapoxvirus* vector according to the invention still exhibits excellent growth behavior and has the capability of expressing trans- or foreign genes. The analyses performed by the inventors demonstrate a high potential for the design of vector-based vaccines.

Due to the identified increased immunogenicity the modified *Parapoxvirus* or *Parapoxvirus* vector according to the invention are also well-qualified for other applications, such as oncolytic virus or vector, immune stimulant, a tool for gene therapy or an inactivated virus, respectively.

The findings of the inventors were surprising and could not be expected by the skilled person. It could be assumed that the ORF encoding the CBP is essential or at least favorable for the functioning and replicability or the *Parapoxvirus* genome and, consequently, for a *Parapoxvirus-based* vector. In other words, it could have been assumed that in a *Parapoxvirus* vector it cannot be dispensed of the ORF encoding the CBP. Furthermore, a functional mutation in a viral ORF typically results in a loss of immunogenicity and it could have been expected the same effect when mutating the ORF encoding CBP in *Parapoxviruses.* The art, therefore, points into a direction opposite to the one the invention points to.

The problem underlying the invention is fully achieved herewith.

In an embodiment of the invention the functional mutation results in a reduction of the activity of CBP over non-mutated CBP.

This embodiment includes any kind of genetic alteration in the ORF encoding CBP which results in a loss of function of CBP (loss-of-function mutation) or, in comparison with the wild type counterpart, a significant reduction of CBP expression or activity, respectively. Therefore, this embodiment encompasses a downregulation of CBP function which need not be a complete inactivation. However, a complete inactivation or cativity reduction to zero of CBP is preferred in an embodiment of the invention. The mutation includes, without being limited thereto, knockout, point, deletion, frame shift, and substitution mutations etc., which all result in the inactivation of CBP function, whether a dysfunctional CBP will be the outcome or the expression of CBP is suppressed, reduced or avoided, respectively.

In an embodiment of the invention the modified *Parapoxvirus* is a *Parapoxvirus ovis (Orf* virus, ORFV) or the *Parapoxvirus* vector is a *Parapoxvirus ovis (Orf* virus, ORFV) vector.

The *Parapoxvirus ovis* or *Orf* virus (ORFV) is the prototype of the genus of the *parapoxviruses* and belongs to the family of the *Poxviridae.* ORFV are enveloped, complex dsDNA viruses having a morphology that reminds of a ball of wool and have an average size of approx. 260 x 160 nm. They comprise a linear DNA genome with high GC content and a size of approx. 130 to 150 kbp, the central region of which is delimited on both sides by ITR regions ("inverted terminal repeats") and ends in a hair-pin structure, which covalently links both DNA single strands to each other. In the central region of the genome there are predominantly genes which are mainly essential for the viral replication and morphogenesis and which are highly conserved among the *poxviruses.* In contrast, in the ITR regions there are so-called non-conserved virulence genes which significantly determine the host range, the pathogenicity and the immunomodulation and, therefore, characterize the virus.

ORFV has a variety of characteristics which makes it interesting for the production of recombinant vaccines and prefers it over other technologies. In comparison to *orthopoxviruses* ORFV is characterized by a very narrow natural host tropism which includes sheep and goats. As a consequence, an inhibiting "preimmunity" against the vector in humans, which is caused by a natural infection, as it can be observed in the most common viral vectors of the *vaccinia* and *adenoviruses,* can be almost excluded. Furthermore, the exceptionally weak and short-lived ORFV specific vector immunity allows a very effective booster and/or refreshing vaccination or immunization with ORFV based vaccines which are directed against further pathogens.

In another embodiment of the invention said ORFV is of the strain D1701, preferably of the strain D1701-V.

This measure has the advantage that such a virus or vector is used which is attenuated and causes only asymptotic infections in the host. According to the invention all variants of D1701 are covered, including D1701-B and D1701-V while the latter is preferred. The characteristics of the strain D1701-V are disclosed in Rziha et al. (2019), Genomic Characterization of Orf Virus Strain D1701-V (Parapoxvirus) and Development of Novel Sites for Multiple Transgene Expression. Viruses 11(2), p. 127.

In another embodiment, said CBP is encoded by viral open reading frame 112 (ORF112), preferably said open reading frame (ORF) is located at the nucleotide positions nt 10.647 ± 100 to nt 11.508 ± 100.

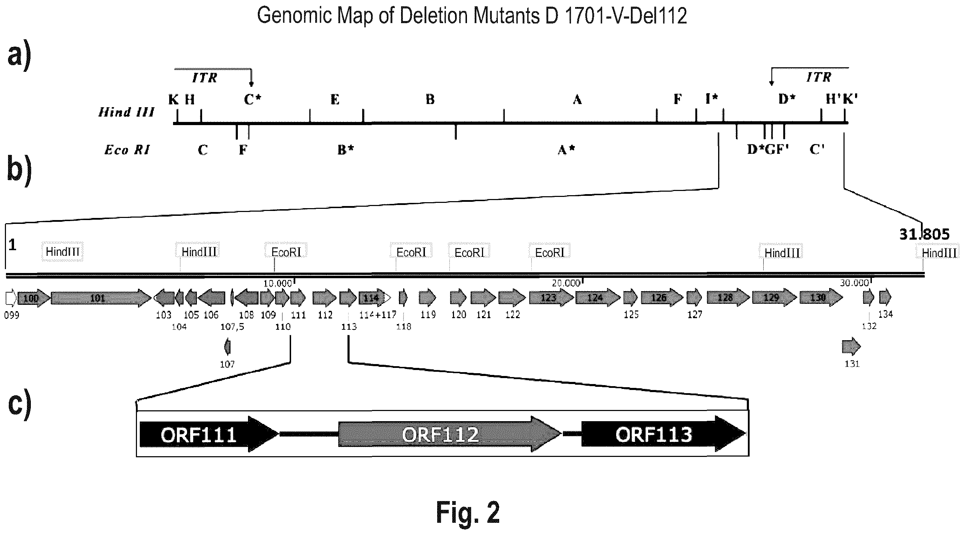

With this measure, the particular open reading frame is provided as the target for inactivation, which in ORFV D1701 encodes the CBP. The skilled person, therefore, receives detailed information of the place of mutation. In this context the indicated nucleotide positions refer to a 31.805 nt comprising DNA sequence encoded by the right hand part of the ORFV D1701-V genome determined by Rziha, H.-J., unpublished data as indicated in Figure 2b.

In a further embodiment the modified *Parapoxvirus* or *Parapoxvirus* vector according to the invention further comprises:
(1) at least one nucleotide sequence encoding a transgene, and
(2) at least one promoter controlling the expression of the transgene-encoding nucleotide sequence.

According to the invention a 'transgene', or synonymously a foreign gene, refers to a gene or open reading frame (ORF) which does not originate from the *Parapoxvirus* genome.

According to the invention a 'promoter' refers to such a nucleic acid section which allows the regulated expression of the transgene in the *Parapoxvirus* vector of the invention. Preferably it refers to an ORFV promoter, i.e. a promoter existing in the wild type ORFV genome or a promoter derived therefrom, or an artificial promoter, such as a *poxvirus* promoter, *CMV* promoter etc.

This measure has the advantage that the virus or vector according to the invention is used as a genetic tool for the production of any foreign protein, such as an antigen. Together with the vector's excellent immunogenicity properties such embodiment improves the suitability of the virus or vector according to the invention as active substance or component of a vaccine composition.

In a yet further embodiment of the invention said transgene-encoding nucleotide sequence is inserted into said CBP-encoding viral ORF, and/or, alternatively, said CBP-encoding viral ORF is replaced by said transgene encoding nucleotide sequence.

According to the invention 'inserted into said CBP-encoding viral ORF' means that the CBP-encoding sequence or open reading frame, respectively, is deleted or opened-up at any position and the transgene-encoding sequence along with its promoter is inserted. Sections of the CBP-encoding sequence may or may not be removed by said procedure. According to the invention 'replaced by said transgene encoding nucleotide sequence' means that the entire CBP-encoding viral ORF is removed from the virus or vector and the sequence gap receives the transgene-encoding nucleotide sequence along with its promoter. This embodiment has the advantage that the insertion of the transgene-encoding nucleotide sequence provides for the functional mutation or inactivation of the CBP.

In another embodiment said modified *Parapoxvirus* or *Parapoxvirus* vector according to the invention comprises more than one transgene-encoding nucleotide sequence, preferably the number of transgene-encoding nucleotide sequences is selected from the group consisting of: 2, 3, 4 or more.

In this embodiment the respective transgenes or transgene-encoding sequences can all be located in the CBP-encoding viral ORF. However, alternatively, the various transgene-encoding sequences can also be located elsewhere at the same or different locations in the virus or vector genome or construct, respectively. In every insertion locus multiple foreign genes, preferably 2, 3, 4 or more, can be expressed. For example, in ORFV D1701or D1701-V the transgene-encoding sequences can preferably be located in any of the viral ORFs 112, 119, 126, etc.

This measure has the advantage that by means of a single modified *Parapoxvirus* or *Parapoxvirus* vector according to the invention multiple foreign or transgenes can be expressed. This embodiment is especially appropriate for the production of polyvalent vaccines which, at the same time, are directed against a number of antigenic structures.

In another embodiment of the modified *Parapoxvirus* or *Parapoxvirus* vector according to the invention the promoter is an early ORF promoter, which preferably comprises a nucleotide sequence which is selected from: SEQ ID NO: 1 (eP1), SEQ ID NO: 2 (eP2), SEQ ID NO: 3 (optimized "early"), SEQ ID NO: 4 (7.5 kDa promoter), and SEQ ID NO: 5 (VEGF).

This measure has the advantage that such promoters are used which allow a high expression level of the transgene and a targeted control of the expression. The promoters eP1 and eP2 were developed by the inventors and are published in Rziha, H.-J., et al. (2019; *l.c.).* The remaining promoters originate from the vaccinia virus and are described in other connections in Davidson and Moss (1989), Structure of Vacciniavirus late promoters, J. Mol. Biol., Vol. 210, pp- 771-784, and Yang et al. (2011), Genome-wide analysis of the 5' and 3' ends of Vaccinia Virus early mRNAs delineates regulatory sequences of annotated and anomalous transcripts, J. Virology, Vol. 85, No. 12, pp. 5897-5909, Broyles (2003), Vaccinia virus transcription, J. Gene. Virol., Vol. 84, No. 9, pp. 2293-2303. According to the findings of the inventors P2 causes a significantly increased expression strength in comparison to eP1. This was surprising because the promoter eP1 corresponds by 100% to the consensus sequence from the *Vacciniavirus,* however not eP2. The low expression of the "optimum" *Vacciniavirus* promoter *(Orthopox)* in ORFV *(Parapox)* is a contradiction and surprising. It is also surprising that the eP2 promoter results in a strong expression.

In another embodiment of the modified *Parapoxvirus* or *Parapoxvirus* vector according to the invention the transgene is selected from the group of the following antigens:
- viral antigen, preferably
   severe acute respiratory syndrome coronavirus 2 (SARS-CoV-2) antigen, including spike (S), envelope (E), and nucleocapsid (N) proteins;
   rabies virus antigen, including glycoprotein (RabG);
   influenza A antigen, including nucleoprotein (NP), hemagglutinin (HA), neuraminidase (NA);
- tumor antigen, preferably viral tumor antigen, including HPV selective viral tumor antigen;
- tumor associated antigen, including viral tumor associated antigen, including HPV selective viral tumor-associated antigen;
- parasitic antigen, preferably plasmodium antigen;
- cytokine;
- protein originated or derived from a mammal, preferably from a mammal recipient.

This measure has the advantage that especially important antigens, in particular for the production of vaccines, are expressible via the modified *Parapoxvirus* or *Parapoxvirus* vector according to the invention.

Another subject matter of the present invention relates to a biological cell, preferably a mammalian cell, further preferably a Vero cell, a HEK 293 cell or an antigen-presenting cell, containing the modified *Parapoxvirus* or *Parapoxvirus* vector according to the invention.

Vero and HEK 293 cells are currently used for the production of the modified virus or vector according to the invention. In the host, however, the virus is been taken up be antigen-presenting cells.

Another subject matter of the present invention relates to a composition, preferably a pharmaceutical composition, containing the modified *Parapoxvirus* or *Parapoxvirus* vector of the invention and/or the cell of the invention, and a pharmaceutically acceptable carrier. The pharmaceutical composition can be preferably a vaccine, further preferably a polyvalent vaccine, an immune stimulant, a tool for gene therapy, etc.

Pharmaceutically acceptable carriers are well known in the art. They include, without being limited thereto, stabilizers, binders, diluents, salts, adjuvants, buffers, lipids, etc. An overview can be found in Rowe (2020), Handbook of Pharmaceutical Excipients, 9th edition, Pharmaceutical Press.

The characteristics, advantages, features, and further developments of the modified *Parapoxvirus* or the *Parapoxvirus* vector according to the invention apply to the cell of the invention and the composition of the invention in a corresponding manner.

Another subject-matter of the invention relates to the use of a modified *Parapoxvirus* or a *Parapoxvirus* vector comprising at least one functional mutation in the viral open reading frame (ORF) encoding the 'chemokine binding protein' (CBP) for the induction of an immune response in a living being, preferably a mammal or a human being.

The characteristics, advantages, features, and further developments of the modified *Parapoxvirus* or the *Parapoxvirus* vector according to the invention apply to the use of the invention in a corresponding manner.

It is to be understood that the previously mentioned features and those to be explained in the following cannot only be used in the combination indicated in each case, but also in other combinations or in isolated position without departing from the scope of the invention.

### EMBODIMENTS

The invention is now further explained by means of embodiments resulting in additional features, characteristics and advantages of the invention. The embodiments are of pure illustrative nature and do not limit the scope or range of the invention. The features mentioned in the specific embodiments are features of the invention and may be seen as general features which are not applicable in the specific embodiment but also in an isolated manner in the context of any embodiment of the invention. Reference is made to the enclosed figures where the following is shown.

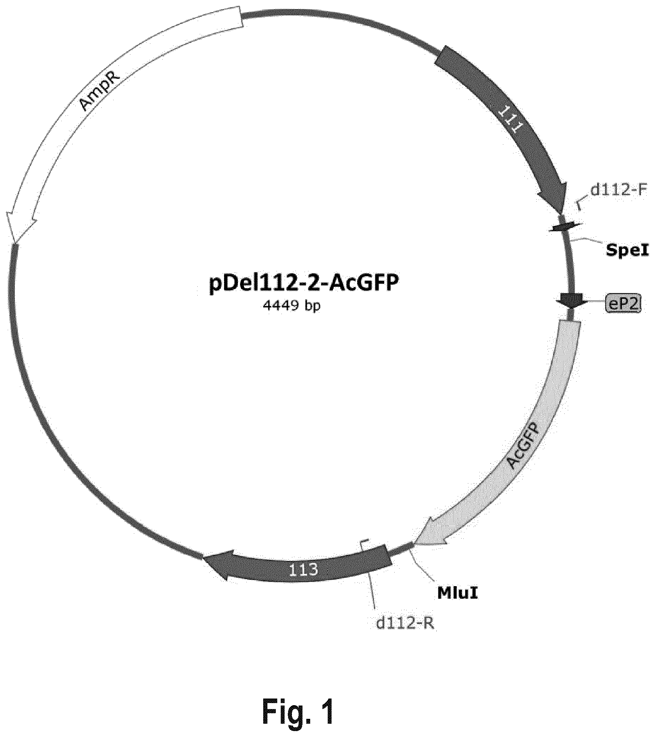

- Fig. 1:: Plasmid chart of pDel112-2-AcGFP.
- Fig. 2:: Graphical description of ORF112 deletion (Del112) in the D1701-V genome. A) Shows the genomic map of ORFV strain D1701-V as recently published by Rziha et al. (2019; *l.c.).* B) Open reading frame (ORF) encoded by the right-hand part of the genome; the 31.805 nt comprising DNA sequence was determined by Rziha, H.-J., unpublished data. C) Enlargements of the genomic sites affected by each gene deletion. The corresponding sequences are given in SEQ ID NO: 6.

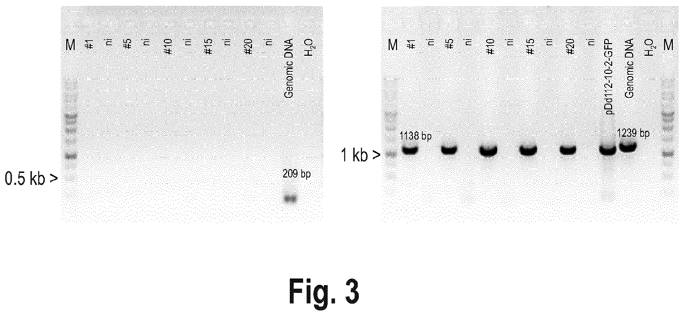

- Fig. 3:: GFP is stably integrated into Del112. 112 PCR after passage 1, 5, 10, 15 and 20 of VCh112GFP in Vero cells indicates a deletion of gene 112 (A), while d112 PCRs result in 1138 bp fragments specific for GFP inserted into the Del112 locus. 1% agarose gel; ni = DNA from not infected Vero cells; M = Ready-To-Use 1 kb Ladder, Nippon Genetics.

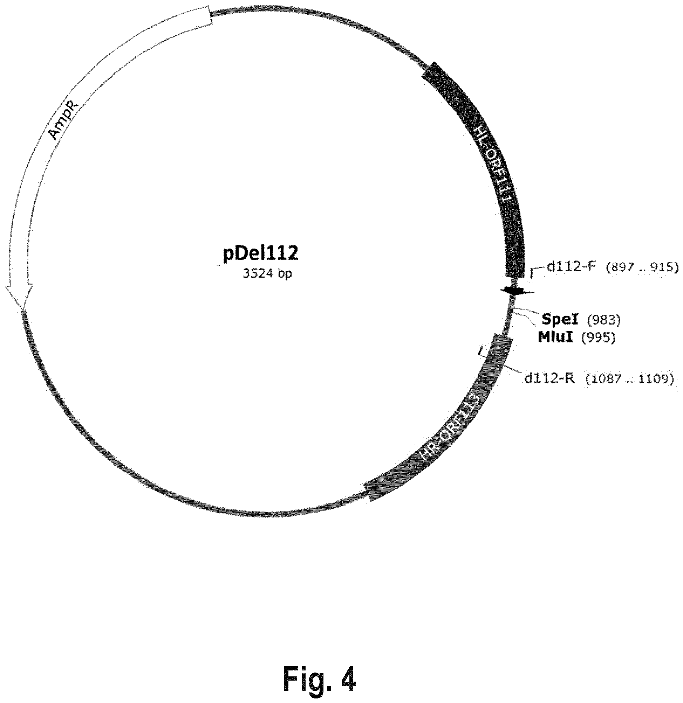

- Fig. 4:: Plasmid chart of pDel112.

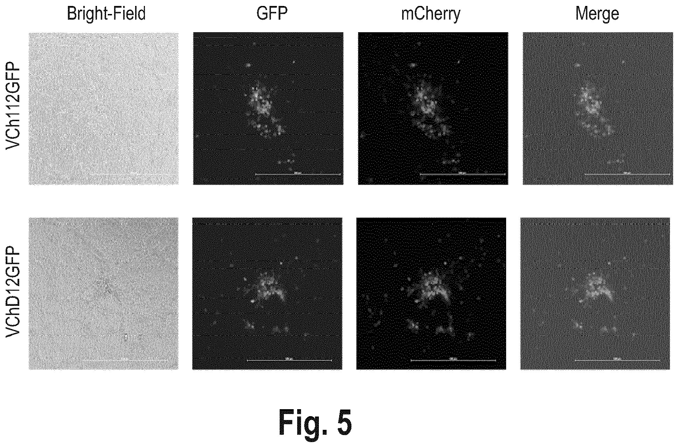

- Fig. 5:: New Del-site recombinant VCh112GFP induces the expression of GFP and mCherry in Vero cells. Fluorescence microscopy of single plaques was performed five days after infection with the new Del-site recombinant and the reference virus VChD12GFP. Pictures obtained from bright field microscopy, single GFP and mCherry channels as well as merged pictures are shown, while scale bars represent 500 µm.

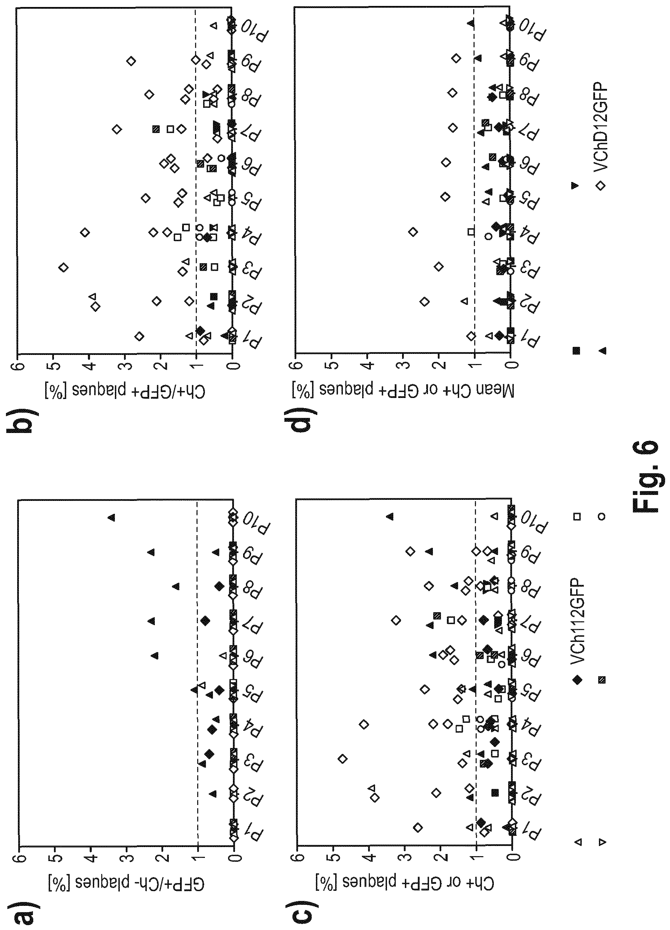

- Fig. 6:: Genetic stability of transgenes gfp and mcherry in Del-site recombinant VCh112GFP. During ten serial passages (P1-P10), the expression of fluorophores by infected Vero cells was determined by single plaque counting after 72 h post infection. Frequencies of single GFP (A) and single mCherry (B) expressing plaques, as well as the overall frequency of plaques showing single fluorescence (C) are shown for three viral clones per Del-site recombinant obtained from 96-well limiting dilutions. The mean overall frequency of single fluorescent plaques was calculated and plotted in (D).

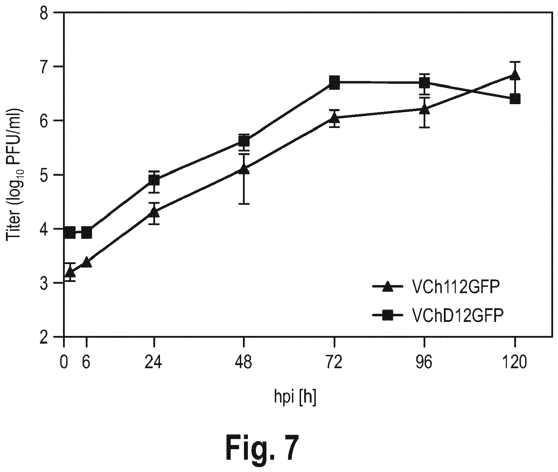

- Fig. 7:: Single step growth curves of Del-site recombinant VCh112GFP. Infected cells (MOI 1) were washed and harvested 2 h after adsorption (0 hpi) and at the indicated hours post infection (hpi), while total cell lysates were titrated on Vero cells to determine the virus titer (PFU/ml). The virus growth curve of the recombinant VCh112GFP reaches comparable virus titers than the control VChD12GFP after 120 h.

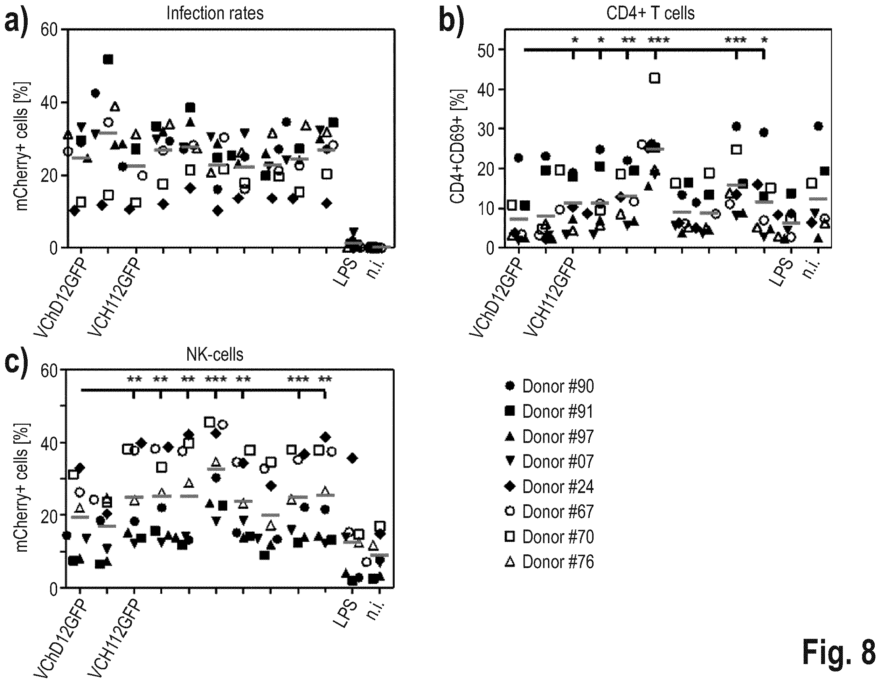

- Fig. 8:: Activation of human PBMCs by new Del-site recombinants. PBMCs of eight different donors were infected for 24 h with VCh112GFP and the reference virus VChD12GFP. The percentage of infected cells was determined by mCherry expression of the CD14+ cell population (A), while the activation of CD4+ (B) and CD56+ NK-cell (C) populations was determined using CD69 as activation marker. Shown are the CD69+ fractions of different PBMC populations for each of the eight independently performed experiments and their means. Statistical analysis was performed using a paired t-test with a confidence interval of 95%: ns = p ≥ 0.05, * = p < 0.05, ** = p < 0.01, *** = p < 0.001.

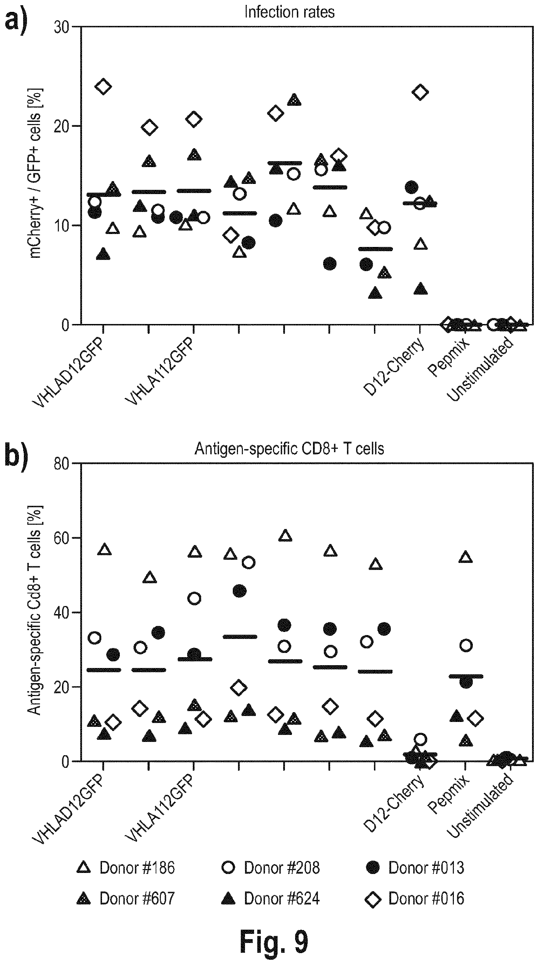

- Fig. 9:: Infection rates and expansion of memory T cells of 6 different donors after infection with the new Del-site recombinant VHLA112GFP. CD14-PBMC populations were stimulated with Pepmix or CD14+ monocytes infected with VHLA112GFP and the reference virus VHLAD12GFP. D12-Cherry infected as well as unstimulated cells served as controls. A) Infection rates were determined 24 hpi by the expression of mCherry or GFP in CD14+ monocytes using FACS. B) After 12 days, the expansion of memory T cells was determined by the proportion of tetramer+ CD8+ T cells (antigen-specific CD8+ T cells). Colors indicate memory T cell specificities possessed by the different donors: Black: GLCTLVAML and GILGFVFTL; Blue: GILGFVFTL and NLVPMVATV; Green: GLCTLVAML, GILGFVFTL and NLVPMVATV. Shown are the results of 6 independent experiments and the calculated mean values. The statistical analysis was performed using a paired t-test with a confidence interval of 95%: ns = p ≥ 0.05, * = p < 0.05, ** = p < 0.01, *** = p < 0.001.

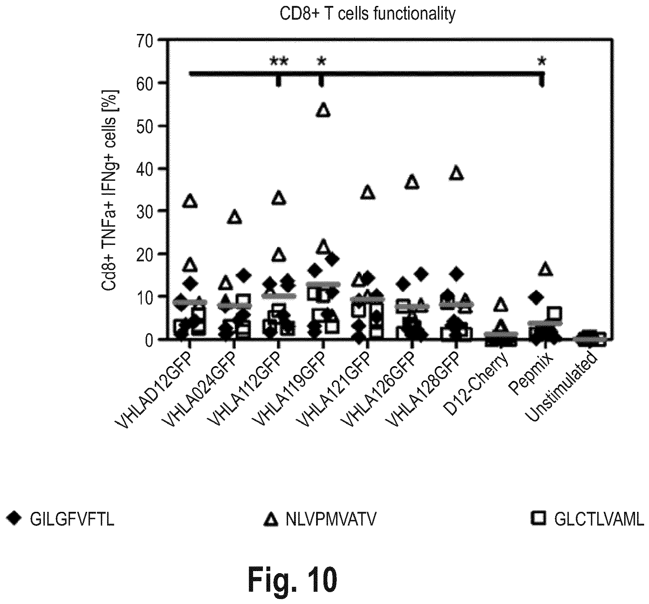

- Fig. 10:: Functionality of memory T cells of 6 different donors after infection with the new Del-site recombinant VHLA112GFP. CD14- PBMC populations were stimulated with Pepmix or CD14+ monocytes infected with VHLA112GFP and the reference virus VHLAD12GFP. D12-Cherry infected as well as unstimulated cells served as controls. The functionality of the expanded CD8+ T cells was analyzed after re-stimulation with GILGFVFTL, GLCTLVAML or NLVPMVATV peptides by intracellular staining of TNFα and IFNγ, and the proportion of TNFα+ IFNγ+ CD8+ T cells was determined. Shown are the results of 6 independent experiments and the calculated mean values. The statistical analysis was performed using a paired t-test with a confidence interval of 95%: ns = p ≥ 0.05, * = p < 0.05, ** = p < 0.01, *** = p < 0.001.

### 1. Preliminary remark

The present invention relates to the use of modified *Parapoxviruses or Parapoxvirus* vectors as a tool for the induction of an increased immune response in living beings and, in a further development, the expression of a trans- or foreign gene. To allow the insertion of multiple transgenes into a single *Parapoxvirus* vector, the present invention also relates to the suitability of the open reading frame (ORF) encoding the 'chemokine binding protein' (CBP), such as ORF112 in *Parapoxvirus ovis (Orf* virus, ORFV) stain D1701and D1701-V, as insertion site for transgene expression. The novel deletion mutants were subjected to detailed characterization of the genetic stability of inserted AcGFP (GFP) reporter constructs, their growth behavior and capability to induce transgene expression in different target cells *in vitro.* Additionally, the mutants' immunogenicity was analyzed by its ability to activate peripheral blood mononuclear cells, or to induce antigen-specific immune responses *in vitro.* Taken together, the analyses performed demonstrate a high potential for the design of polyvalent, single vectored vaccines by integrating knockouts of CBP-encoding ORFs into the *Parapoxvirus* genome. Thus, the exchange of the open reading frame with the reporter gene GFP resulted not only in efficient replication of stable vectors expressing the desired transgene, but also attributed remarkable immunogenicity properties to the newly generated recombinants.

### 2. Introduction

The ORFV strain D1701-V was obtained from the bovine kidney cell line BK-KL3A adapted strain D1701-B and showed several genomic rearrangements after adaptation for growth in the African green monkey cell line Vero; Cottone, R., et al. (1998), Analysis of genomic rearrangement and subsequent gene deletion of the attenuated Orf virus strain D1701, Virus Research 56(1), p. 53-67; Rziha, H.J., et al. (2000), Generation of recombinant parapoxviruses: non-essential genes suitable for insertion and expression of foreign genes, Journal of Biotechnology 83(1), p. 137-145. These genomic rearrangements include the deletion of genes that are non-essential for the strain's replication and were shown suitable for transgene expression such as the deleted region D (D locus); Rziha, H.-J., et al. (2019; *l.c.).* Furthermore, the angiogenic factor VEGF-E was predicted a major virulence determinant responsible for the induction of bloody lesions in sheep Rziha, H.J., et al. (2000; *l.c.)* and thus, used as insertion site to generate ORFV recombinants triggering long-lasting immunity with a high protective efficacy against several viral diseases in different hosts. Besides these successfully used insertion sites, other genes potentially suited for transgene expression have been identified in the terminal ends of the D1701-V genome. As the central regions of poxviruses generally encode for essential genes conserved in position, spacing and orientation, these regions encode for factors influencing virulence, pathogenesis or host range and are considered dispensable for *in vitro* growth. While these factors are predicted to interfere with the optimal induction of cellular and humoral immune responses triggered by the host, a deletion of these immunomodulatory genes may further enhance the immunogenicity of the D1701-V vector. Thus, the present work focuses on the deletion of the gene encoding the chemokine binding protein (CBP) and its use as insertion site for transgene expression.

### Chemokine binding protein (CBP) - ORF112

The family of chemokines consists of secreted chemotactic proteins that activate and regulate the homeostatic migration and recruitment of leukocytes to sites of infection through the lymphoid organs. The classification into CC, CXC, C or CX3C chemokines relies on their N-terminal cysteine arrangement, which impacts the cell type migrating along the chemokine gradient towards the site of inflammation. Hence, CC chemokines generally attract monocytes, while lymphocytes, NK and B cells as well as neutrophils sense CXC chemokines. To attenuate inflammation of infected cells, the *Orthopoxvirus* and *Leporipoxvirus* genera produce the poxvirus type II CC-chemokine binding proteins (CBP-II) that share a 17% sequence identity to the CBP encoded by ORFV (ORF112). This CBP has been shown to be functionally similar to the poxviral CBP-II in its ability to bind lymphotactin or many human inflammatory and constitutive CC-chemokines such as CCL19 or CCL21 with high affinity and thus, to play a critical role in virulence and pathogenesis; Counago, R.M., et al. (2015), Structures of Orf Virus Chemokine Binding Protein in Complex with Host Chemokines Reveal Clues to Broad Binding Specificity, Structure 23(7), p. 1199-213; Lateef, Z., et al. (2010), The chemokine-binding protein encoded by the poxvirus orf virus inhibits recruitment of dendritic cells to sites of skin inflammation and migration to peripheral lymph nodes, Cell. Microbiol. 12(5), p. 665-76; Seet, B.T., et al. (2003), Analysis of an orf virus chemokine-binding protein: Shifting ligand specificities among a family of poxvirus viroceptors, Proc. Natl. Acad. Sci. USA, 100(25), p. 15137-42; Fleming, S.B., et al. (2017; *l.c.).*

### 3. Material and methods

### Generation and characterization of new Del-site recombinants

In order to generate the ORFV recombinants used in this work, transgenes synthesized and obtained from Invitrogen were cloned into transfer plasmids. Stable integration of transgenes into the ORFV genome was accomplished by homologous recombination between the transfer plasmid and the genomic DNA of the parental virus following transfection by nucleofection and subsequent infection of Vero cells.

### Generation of transfer plasmids

DNA inserts as well as plasmid vectors were digested using the same restriction enzymes, fragments of interest were purified and ligated. Subsequently, bacteria were transformed with ligated plasmids and colonies selected to produce the transfer plasmids. For validation, control digests using restriction enzymes was followed by agarose gel electrophoreses, while DNA sequencing using insert specific primers confirmed correct insertion of the transgenes into the plasmid vector. The following table summarizes the transfer plasmid generated, while the plasmid chart and corresponding sequence (SEQ ID NO: 7) are shown in Figure 1.

| **Transfer Plasmid** | **Insert Vector** | **Parental Vector** | **Restriction digest** |
|---|---|---|---|
| **pDel112-2-AcGFP** | pD12-AcGFP | pDel112 | Mlu/Spe/ (937+3512 bp) |

### Transfection of ORFV infected Vero Cells

The generation of recombinant ORFVs followed two steps, in which Vero cells were transfected with transfer plasmids prior to infection with ORFV. Transfection was performed by an electroporation-based transfection method called nucleofection. Due to homologous sequences on the transfer plasmid and the ORFV genome, the inserts could be integrated into the ORFV genome via homologous recombination. For this purpose, Vero cells were detached using trypsin, re-suspended in 5 ml NF stop solution and counted. For each transfection batch, 2.5 x 10⁶ cells were transferred into a 1.5 ml Eppendorf cup and centrifuged at 63 rcf for 10 min. The cell pellet was re-suspended in 100 µl transfection solution (transfection supplement and CLB transfection buffer of the Amaxa transfection kit mixed in a ratio of 1:4.5), supplemented with 2 µg plasmid DNA and transferred into a transfection cuvette. For transfection, the CLB Transfection Device was used. Subsequently, nucleofected cells were re-suspended in NF stop solution and transferred into a T25 cell culture flask containing 6 ml pre-warmed Vero cell medium using a Pasteur pipette. Cells were immediately infected with parental virus (MOI 1) for 4 hours at 37°C and 5% CO₂, washed using 6 ml pre-warmed PBS and incubated in fresh Vero cell medium for 72 h at 37°C and 5% CO₂. When virus plaques or a cytopathic effect

(CPE) could be observed, cells were frozen and thawed three times at -80°C and 37°C in a water bath, respectively, to disrupt the cells and release viral progeny.

### Selection of ORFV recombinants

Homologous recombination is an event occurring in a ratio of approximately 1:10000 between the insert of the transfer plasmid and the target region in the ORFV genome. To select for this rare event and to separate the recombinant ORFVs from parental viruses, different selection methods can be used.

### FACS based selection of ORFV recombinants

Selection by fluorescence activated cell sorting (FACS) is based on the loss or gain of a fluorescent label such as GFP or mCherry. For this purpose, 3 x 10⁵ Vero cells were seeded in a 6-well plate containing 3 ml Vero cell medium. Transfection lysate was used for infection in serial dilutions and took place for 20-24 h at 37°C and 5% CO₂. To ensure an optimal selection process, cells with a low infection rate of approximately 1-5% were harvested and centrifuged for 5 min at 400 rcf. Subsequently, cells were washed thrice with 1 ml PBE and eventually re-suspended in 500 µl PBE. Single cell FACS sorting was performed into a 96-well plate containing 10⁴ Vero cells per well in 150 µl Vero cell medium using the BD FACSjazz (Biosciences). After 72 h of incubation at 37°C and 5% CO₂, wells showing single virus plaques of recombinant ORFV could be picked for further propagations and analyses.

### Selection of ORFV recombinants by limiting dilutions

Further selection and purification of recombinant viruses after FACS based sorting or MACS selection was carried out by limiting dilutions. For this purpose, 2 x 10⁶ Vero cells in 25 ml of Vero cell medium were split into a 12-well pipetting reservoir, in which the first and the rest of wells contained either 3 ml or 2 ml of the cell solution, respectively. The first well was supplemented with either 3 x 10⁵ cells of the MACS selection or 50-100 µl of virus lysate and diluted 1:3 each from the first to the last well. 150 µl of each dilution were transferred into the corresponding wells of a 96-well plate and incubated at 37°C and 5% CO₂ for 72 h. After 72 h, wells containing single virus plaques could be selected for further processing by (fluorescence-) microscopy.

### Determination of genetic stability by serial passages

To investigate the genetic stability of fluorophore coding sites, ten serial passages of respective ORFV recombinants were performed. For this, 5 x 10⁵ Vero cells were seeded in 6-well plates containing 3 ml Vero cell medium. At first, virus lysates showing one single plaque in a limiting dilution were used for infection in serial dilutions and took place for 2 h at 37°C and 5% CO₂. Cells were washed twice with PBS and were subsequently incubated in 3 ml Vero cell medium for 72 h at 37°C and 5% CO₂. Fluorescent plaque counts were determined for wells showing 100-200 plaques using the Eclipse Ti2 microscope (Nikon). Next, cells were frozen at -80°C, thawed at RT and 50 µl of virus lysates were used to infect freshly seeded Vero cells as described above.

### Isolation of PBMCs from donated blood

Peripheral blood mononuclear cells (PBMCs) could be isolated from blood donations obtained from the blood donation center Tübingen. First, the blood was diluted with PBS to a total volume of 100 ml. Next, 4 x 15 ml Ficoll in 50 ml tubes were overlaid with 25 ml of diluted blood each and centrifuged at 700 x g and RT for 20 min. Leukocytes, which could be identified by the formation of a white layer after density gradient centrifugation, were transferred into two 50 ml tubes and each were supplemented with PBS up to a volume of 50 ml. After centrifugation at 400 x g and RT for 10 min, the cell pellets were re-suspended in 50 ml PBS and centrifuged at 300 x g and RT for 10 min. PBMCs were merged and supplemented with PBS up to a volume of 50 ml, and the cell number was determined.

### Isolation of Monocytes from PBMCs

The principle of isolating monocytes from PBMCs relies on the expression of CD14 by monocytes. Hence, PBMCs were centrifuged at 300 x g and RT for 10 min and the pellet was re-suspended in 4 ml PBE and 100 µl of α-CD14 MicroBeads. The suspension was incubated at 4°C for 15 min and loaded onto a LS column equilibrated with PBE. The column was washed three times with 3 ml PBE before the CD14+ monocytes could be eluted from the column with 5 ml PBE and counted.

### Cultivation of monocytes and PBMCs

Monocytes and PBMCs were cultivated in IMDM complete medium at 37°C and 5% CO₂. For experimental procedures, 3 x 105 monocytes and 5 x 10⁵ PBMCs were seeded in 200 µl per well in 96-well round bottom plates, while 1 x 10⁵ monocytes were seeded to be differentiated into dendritic cells.

### Expansion of human antigen-specific memory CD8+ T cells (12 day stimulation)

The expansion of antigen-specific memory CD8+ T cells is based on their stimulation by monocytes presenting HLA-A*02 restricted *Epstein-Barr Virus* (EBV) BMLF1280-288 GLCTLVAML, *Influenza* A MP58-66 GILGFVFTL and *Human Cytomegalovirus* (HCMV) pp65495-503 NLVPMVATV epitopes over a time period of 12 days. Thus, blood from HLA*A02 positive donors was processed as described above and monocytes were cultivated to be infected with various dilutions of different ORFV recombinants or stimulated with 1 µM synthetic peptide, respectively. Additionally, viable cell counting was performed for the CD14- fraction, 5 x 10⁵ cells were seeded in 100 µl T cell medium per well in a 96-well round bottom plate and incubated at 37°C and 5% CO₂. After 20-24h, 20 µl of each infected monocyte group was transferred into a new 96-well plate and washed three times with PFEA by centrifugation at 700 x g and RT. FACS analysis was performed to determine the infection rates, while monocytes showing an infection rate of approximately 20% were co-cultured with CD14- fractions in a total volume of 200 µl T cell medium. Every 2-3 days, 50 µl of medium was replaced by fresh T cell medium containing 20 U/ml IL-2. After 12 days of stimulation, expansion of CD8+ T cells was analyzed by tetramer staining and functionality of the antigen-specific CD8+ T cells was evaluated by intracellular cytokine staining.

### Flow cytometry

The viability and infection rates, as well as the expression of surface and intracellular molecules was analyzed using the BD LSRFortessa flow cytometry system (BD Biosciences). The preparation of samples and staining of cells with fluorescence-labeled antibodies was carried out in 96-well U-bottom plates, while centrifugation occurred at 4°C and 400 x g for 5 min. For the determination of infection rates, cells were washed twice in 200 µl PFEA, and were re-suspended in 50 µl PFEA or optionally fixed for FACS analyses.

### Inhibition of Fc receptors

To prevent non-specific antibody binding, cells expressing Fc receptors were treated with Fc-block according to manufacturers' instructions prior to staining procedures.

### Staining with multimeres

Multimeres are frequently used to identify and quantify antigen-specific T cells. Tetramers consist of four recombinant MHC molecules conjugated to a fluorescently labeled streptavidin complex. In contrast, dextramers consist of a dextran backbone that carries several fluorophores and MHC molecules. Multimeres can be loaded with peptides of interest to form peptide-MHC complexes recognized by specific T cells, and thus, be detected by flow cytometry. For this, cells were washed twice with 200 µl of PBS and re-suspended in 50 µl of tetramer solution or PBS, respectively. Prior to staining with 50 µl of tetramer solution, PE-conjugated HLA tetramers were mixed 1:50 with tetramer buffer and centrifuged at 13.000 x g for 10 min. Staining with dextramers followed the same procedure by mixing dextramers in a ratio of 1:10 with PBS. The incubation was carried out at RT for 30 min in the dark.

### Determination of cell viability

Cell viability was determined by Zombie Aqua staining. Zombie Aqua is an amine-reactive fluorescent dye that cannot penetrate live cells but enters cells with compromised membranes. Therefore, it can be used to differentiate between live and dead mammalian cells. For Zombie Aqua staining, cells were washed twice with 200 µl PBS and re-suspended in 50 µl of Zombie Aqua diluted 1:400 in PBS for 30 min at 4°C in the dark.

### Extracellular antibody staining

For the analysis of surface molecule expression, cells were washed twice with 200 µl PFEA, re-suspended in 50 µl of freshly prepared antibody mix in PFEA and incubated for 30 min at 4°C in the dark.

### Intracellular cytokine staining

For the detection of intracellular cytokines, the cells were treated with 10 µg/ml Brefeldin A to prevent secretion of proteins and stimulated with respective synthetic peptides for 12-14 h. Prior to intracellular cytokine staining, cells were washed twice with 200 µl PFEA and permeabilized by incubation with 50 µl Cytofix/Cytoperm for 30 min at 4°C in the dark. Next, cells were re-suspended and washed twice with 200 µl Permwash and subsequently stained with 50 µl antibody mix in Permwash for 30 min at 4°C in the dark.

### Fixation of cells

For storage exceeding 4 h, cells were fixed. For this, cells were washed twice with 200 µl PFEA, re-suspended in 50 µl PFEA + 1% formaldehyde and stored at 4°C in the dark. Analysis of samples was carried out within 10 days.

### 4. Results

### Generation of new Del-site recombinants

After the successful generation of transfer plasmids, the new ORFV recombinant was generated as described in the methods in detail. Thus, the plasmid pDel112-2-AcGFP was transferred into Vero cells by nucleofection, which were subsequently infected with the parental virus V12-Cherry. Due to homologous sequences in the ORFV genome and the insert flanking regions of the transfer plasmid, homologous recombination led to stable exchange of ORF112 by GFP in the ORFV D1701-V genome (Figure 2). The exact position of ORF112 in the right-hand part of the 31.805 nt comprising DNA sequence of the D1701-V (Rziha, H.-J., unpublished, Figure 2B) is shown in the following table:

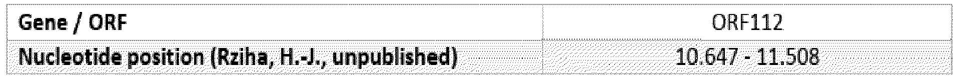

Next, new ORFV recombinants could be isolated from infected GFP and mCherry expressing Vero cells selected by FACS sorting and limiting dilutions. DNA isolation followed by insert and locus specific PCR typing allowed monitoring of the genetic homogeneity of the purified ORFV recombinants as shown in Figure 3. Here, 112 and d112 PCR proved both, the deletion of the ORF112 and exchange with the GFP encoding sequence. Subsequently, the new ORFV recombinants were propagated in large scale as described above.

Recombinant ORFV deletion mutants could also be generated, which only led to the deletion of ORF112 without the insertion of GFP and thus, a loss of functional ORF112 gene product leading to enhanced immunogenicity compared to the parental virus. The corresponding transfer plasmid and sequence can be found in Figure 4 and SEQ ID NO: 8.

### GFP is stably inserted into the new Del-sites

To investigate the stability of inserted GFP into the new Del-sites, Vero cells were infected with the new Del-site recombinant VCh112GFP and the reference virus VChD12GFP and 20 serial passages were performed. Both, GFP and mCherry fluorescence could be observed by fluorescence microscopy in single plaques resulting from Vero cell infection with the new Del-site recombinant throughout this experiment as shown in Figure 5.

Additionally, viral DNA was isolated from infected Vero cells after Passage 1, 5, 10, 15 and 20, and deleted gene-specific as well as locus-specific PCRs were performed to examine the Del-locus' integrity. As shown, no ORF112 specific 209 bp fragments could be detected in 112 PCRs during 20 passages (Figure 3A), while the deletion site showed 1138 bp fragments specific for GFP inserted into the Del112 locus using the d112 PCR (Figure 3B).

Finally, the stability of transgenes inserted into the new Del-site was examined during ten serial passages of three biological replicates (virus clones) of the ORFV D1701-V recombinant. After 72 h of infection, wells containing 100-200 plaques were used to determine the amount of single plaques expressing both, GFP and mCherry fluorophores, or either GFP or mCherry by fluorescence microscopy. The percentages of plaques expressing only one fluorophore during ten serial passages are shown in Figure 6.

The results demonstrate frequencies of less than 1% single GFP and mCherry expressing plaques using VCh112GFP. Taken together, the results presented suggest that GFP is stably expressed in Vero cells infected with the new Del-site recombinant and can be detected throughout 20 passages in the predicted genomic locus. Furthermore, the genetic stability of both, GFP and mCherry in the new Del-site or the vegf-locus, respectively, was validated in ten serial passages studying the fluorescence of infected Vero cells. Since at least 99 % of all counted lytic plaques infected with each new Del-site recombinant expressed GFP and mCherry simultaneously after ten passages, these results indicate genetic stability of the examined genomic loci.

### Characterization of new Del-site recombinants'growth behavior

To investigate, whether the deletion introduced into the new Del-site recombinant alters the *in vitro* growth characteristics in the ORFV permissive Vero cells compared to the reference virus VChD12GFP, single-step growth curve experiments were performed. For this, Vero cells were infected with the new Del-site recombinant VCh112GFP leading to infection rates of approximately 20-25% after 24 h. Infected cells were washed and harvested 2 h after adsorption (designated 0h) or 6 h, 24 h, 48 h, 72 h, 96 h and 120 h post infection. Virus lysates were titrated on Vero cells to determine the number of infectious particles measured in plaque forming units per ml (PFU/ml). The experiment was performed three times for each recombinant and the results are shown in Figure 7-I.

The growth characteristics of Del-site recombinant VCh112GFP resembles the ones obtained for the reference virus VChD12GFP reaching comparable final titers of approximately 10⁷ PFU/ml after 120 hpi. Notably, the growth curve of VCh112GFP indicates lower virus input as used for the growth curve of VChD12GFP and showed only slightly diminished final virus titers compared to the control throughout the experiment (Figure 7). Nevertheless, these results suggest VCh112GFP to be replication efficient as it was able to produce infectious progeny in the permissive cell line Vero.

### Activation of human PBMCs by Infection with new Del-site recombinants

Previously, Müller et al. (2019, in preparation) could show that the uptake of D1701-V ORFV recombinants encoding different transgenes can alter the activation of antigen presenting cells (APCs). Thus, PBMCs were isolated from blood of eight healthy donors and in vitro infected for 24 h with the new Del-site recombinant VCh112GFP and the reference virus VChD12GFP. As monocytes are the only cell population within PBMCs to take up ORFV D1701-V, infection rates were determined by the mCherry expression in the CD14+ PBMC population. Samples showing most comparably infected monocytes within one donor were analyzed. After 24 h, PBMCs were harvested and the expression of the activation marker CD69 was determined on CD4+ T cells and CD56+ NK cells by flow cytometry using surface marker specific antibodies.

Analyses on PBMC activation upon infection using the VCh112GFP and VChD12GFP as a reference is shown in Figure 8. Here, significantly larger populations of CD4+ T cells were activated in PBMCs infected with VCh112GFP compared to the group infected with VChD12GFP. Also the CD56+ NK-cell population revealed significantly stronger activation upon infection with VCh112GFP in comparison to the PBMCs infected with VChD12GFP.

### In vitro expansion of human antigen-specific memory CD8+ T cells

To further study the immunogenicity of the new Del-site recombinant VCh112GFP *in vitro,* its capacity to induce antigen-specific immune responses was analyzed. Therefore, the new Del-site recombinant encoding for the HLA-A*02 restricted EBV (BMLF₁₂₈₀₋₂₈₈ GLCTLVAML), Influenza A (MP₅₈₋₆₆ GILGFVFTL) and HCMV (pp65₄₉₅-₅₀₃ NLVPMVATV) epitopes was generated. Subsequently, CD14+ fractions isolated from human PBMCs were infected with the newly generated recombinant VHLA112GFP and the reference virus VHLAD12GFP or stimulated with a mix of corresponding synthetic peptides (Pepmix). Infection rates were determined by the mCherry or GFP expression using FACS, and monocytes showing comparable percentages within one donor were co-cultured with the CD14-fractions of PBMCs after 24 hpi. The expansion of antigen-specific CD8+ T cells as well as their functionality was determined 12 days after infection via tetramer staining and intracellular cytokine staining (ICS), respectively. Unstimulated CD14-fractions served as negative controls, while stimulation using a mixture of synthetic GLCTLVAML, GILGFVFTL and NLVPMVATV peptides represented the positive control. Additionally, CD14-fractions were stimulated with D12-Cherry infected CD14+ cells to study the influence of ORFV D1701-V without antigen on the expansion and functionality of T cells.

24 h after infection, monocytes showed mean infection rates of approximately 10% - 15%, however, high variations were seen between donors resulting in percentages ranging from 5% - 25% (Figure 9A). After 12 days, the expansion of T cells was measured by tetramer staining and their functionality was analyzed by ICS. Tetramer staining revealed that ORFV D1701-V recombinants encoding the HLA-A*02 restricted EBV, Influenza A and HCMV peptides, or the stimulation with Pepmix elicited antigen-specific CD8+ T cell proliferation. Interestingly, the expansion of CD8+ T cells could be induced against all peptides that the donors possessed memory T cells against. The mean frequencies of CD8+ T cells specific for any of the peptides as well as those obtained for each donor after 12 days of expansion are shown in Figure 9B. A high variation in T cell responses from different donors could be detected. Thus, donor #186 showed antigen-specific CD8+ T cell frequencies of approximately 55% after stimulation, while only 10% CD8+ T cells could be expanded for donor #016. Nevertheless, the new Del-site recombinant as well as the Pepmix induced comparable mean memory T cell responses of approximately 25% than the reference virus VHLAD12GFP, while no antigen specific T cell expansion was detected in the unstimulated or D12-Cherry infected groups. Notably, the responses elicited by the deletion mutant VHLA112GFP were in mean slightly increased compared to the reference virus VHLAD12GFP.

Analyses on the functionality of memory T cells after re-stimulation with GLCTLVAML, GILGFVFTL or NLVPMVATV peptides indicate the production of the proinflammatory cytokines TNFα and IFNγ in approximately 5% of the CD8+ T cells stimulated with Pepmix (Figure 10). Here, the functionality of CD8+ T cells was significantly reduced 2-fold compared to those stimulated by monocytes infected with the reference virus VHLAD12GFP, which showed a mean polyfunctional CD8+ T cell proportion of 10%. Remarkably, the functionality of T cells stimulated with VHLA112GFP was significantly enhanced compared to T cells stimulated with the reference virus VHLAD12GFP.

In conclusion, the immunogenicity of VHLA112GFP encoding for HLA-A*02 restricted EBV, Influenza A and HCMV epitopes was analyzed in vitro by their capacity to induce the expansion of functional CD8+ memory T cells after 12 days. Taken together the results presented above, frequencies of antigen-specific CD8+ T cells could be raised by the stimulation of T cells with monocytes previously infected with the Del-site recombinant VHLA112GFP. Consistently, also the functionality of these T cells in mean showed high polyfunctionality measured by the simultaneous expression of the proinflammatory cytokines TNFα and IFNγ. Thus, the deletion of ORF112 might indicate a positive influence on the strength of antigen-specific cellular immune responses elicited by ORFV 1701-V recombinants.

### 5. Summary

Taken together, the analyses on the new Del-site (CBP) recombinants performed demonstrate a high potential for the design of polyvalent, single vectored vaccines by integrating an ORF112 knockout into the ORFV D1701-V genome. The deletion of ORF112 and simultaneous integration of the reporter construct GFP into the respective Del-site resulted not only in efficient replication of stable vectors expressing the desired transgenes, but also attributed remarkable immunogenicity properties to the newly generated recombinants. Accordingly, the deletion of ORF112 is abolishing the ORFV induced expression of a CBP, which was shown to bind several inflammatory CC-chemokines such as MCP-1, MIP-1α or RANTES and found to be expressed by moDCs and PBMCs infected with recombinant ORFV D1701-V; Seet, B.T., et al. (2003), Analysis of an orf virus chemokine-binding protein: Shifting ligand specificities among a family of poxvirus viroceptors, Proc. Natl. Acad. Sci. USA 100(25), p. 15137-42; Müller, M. (2019), Weiterentwicklung des Orf-Virus Stamms D1701-V zur Verwendung als therapeutische Vektor-Vakzine, in Interfaculty Institute of Cell Biology, Department of Immunology, Dissertation Eberhard Karls Universität Tübingen. Furthermore, its deletion was shown to strongly reduce the virulence and pathogenesis of ORFV in sheep, which contributes significantly to safety aspects of the viral vector, and to favor the influx of NK cells, monocytes/macrophages and granulocytes to the site of infection; Fleming, S.B., et al. (2017; *I.c*.); Graham, K.A., et al. (1997), The T1/35kDa family of poxvirus-secreted proteins bind chemokines and modulate leukocyte influx into virus-infected tissues, Virology 229(1): p. 12-24. In addition to binding CC-chemokines, the interaction of CBP with the C-chemokine lymphotactin involved in the chemotaxis of T cells, neutrophils and B cells was reported; Huang, H., et al. (2001), Neutrophils and B cells express XCR1 receptor and chemotactically respond to lymphotactin, Biochemical and Biophysical Research Communications 281(2): p. 378-382; Kelner, G.S., et al. (1994), Lymphotactin - a Cytokine that represents a new class of chemokine, Science, 266(5189): p. 1395-1399; Yoshida, T. et al. (1998), Identification of single C motif-1 lymphotactin receptor XCR1, Journal of Biological Chemistry 273(26): p. 16551-16554.

Therefore, the lack of CBP might constitute an important factor driving especially the B cell responses which could be displayed as increased antigen-specific antibody titers in *in vivo* experiments. Thus, the findings of the present invention may pave the way for the generation of ORFV D1701-V vectored vaccines with favorable properties, in which recombinants harboring CBP or ORF112 deletions simultaneously expressing several antigens and immunomodulatory elements lead to the induction of strong, long-lasting and efficient cellular as well as humoral immune responses.

### 6. Sequences

| | |
|---|---|
| SEQ ID NO: 1: | Nucleotide sequence of eP1 promoter |
| SEQ ID NO: 2: | Nucleotide sequence of eP2 promoter |
| SEQ ID NO: 3: | Nucleotide sequence of "optimized early" promoter |
| SEQ ID NO: 4: | Nucleotide sequence of 7.5 kDa promoter |
| SEQ ID NO: 5: | Nucleotide sequence of VEGF promoter |
| SEQ ID NO: 6: | Nucleotide sequence of D1701-V ORF111 - 112 -113 (nt 1 - 2331) |
| | ORF111: nt 1 - 537 |
| | ORF112: nt 766 - 1626 |
| | ORF113: nt 1702-2328 |
| SEQ ID NO: 7: | Nucleotide sequence pDel 112-2-AcGFP (nt 1 - 2057) |
| | HL (homolog left arm) ORF111: nt 1 - 519 |
| | Early promoter eP2: nt 722 - 758 |
| | AcGFP: nt 790 - 1506 |
| | HR (homolog right arm) ORF113: nt 1580 - 2057 |
| SEQ ID NO: 8: | Nucleotide sequence pDel112 (nt 1 - 1132) |
| | HL (homolog left arm) ORF111: nt 1 - 519 |
| | HR (homolog right arm) ORF113: nt 655 - 1132 |

## Claims

1. A modified *Parapoxvirus* comprising at least one functional mutation in the viral open reading frame (ORF) encoding the 'chemokine binding protein' (CBP), wherein said virus comprises increased immunogenicity in comparison with the same vector without said functional mutation.

2. The modified *Parapoxvirus* of claim 1, wherein said functional mutation results in a reduction of the activity of CBP over non-mutated CBP.

3. The modified *Parapoxvirus* of claim 1 or 2, which is a *Parapoxvirus* vector.

4. The modified *Parapoxvirus* or *Parapoxvirus* vector of any of the preceding claims, wherein the *Parapoxvirus* is a *Parapoxvirus ovis (Orf* virus, ORFV) or the *Parapoxvirus* vector is an ORFV vector.

5. The modified *Parapoxvirus* or *Parapoxvirus* vector of claim 4, wherein said ORFV is of the strain D1701, preferably of the strain D1701-V.

6. The modified *Parapoxvirus* or *Parapoxvirus* vector of any of the preceding claims, wherein said CBP is encoded by viral open reading frame 112 (ORF112), preferably said ORF is located at the nucleotide positions nt 10.647 ± 100 to nt 11.508 ± 100.

7. The modified *Parapoxvirus* or *Parapoxvirus* vector of any of the preceding claims, further comprising:
(1) at least one nucleotide sequence encoding a transgene, and
(2) at least one promoter controlling the expression of the transgene-encoding nucleotide sequence.

8. The modified *Parapoxvirus* or *Parapoxvirus* of claim 6, wherein said transgene-encoding nucleotide sequence is inserted into said CBP-encoding viral ORF, or/and wherein said CBP-encoding viral ORF is replaced by said transgene-encoding nucleotide sequence.

9. The *Parapoxvirus* vector of any of claims 6-8, comprising more than one transgene-encoding nucleotide sequence, preferably the number of transgene-encoding nucleotide sequences is selected from the group consisting of: 2, 3, 4 or more.

10. The modified *Parapoxvirus* or *Parapoxvirus* vector of any of claims 6-9, wherein the promoter is an early ORFV promoter.

11. The modified *Parapoxvirus* or *Parapoxvirus* vector of claim 10, wherein the early ORFV promoter comprises a nucleotide sequence which is selected from the group consisting of: SEQ ID NO: 1 (eP1), SEQ ID NO: 2 (eP2), SEQ ID NO: 3 ("optimized early"), SEQ ID NO: 4 (7.5 kDa promoter), and SEQ ID NO: 5 (VEGF).

12. The modified *Parapoxvirus* or *Parapoxvirus* vector of any of the preceding claims, wherein the transgene is selected from the group of the following antigens:
- viral antigen, preferably
severe acute respiratory syndrome coronavirus 2 (SARS-CoV-2) antigen, including spike (S), envelope (E), and nucleocapsid (N) proteins;
rabies virus antigen, including glycoprotein (RabG);
influenza A antigen, including nucleoprotein (NP), hemagglutinin (HA), neuraminidase (NA);
- tumor antigen, preferably viral tumor antigen, including HPV selective viral tumor antigen;
- tumor associated antigen, including viral tumor associated antigen, including HPV selective viral tumor-associated antigen;
- parasitic antigen, preferably plasmodium antigen;
- cytokine;
- protein originated or derived from a mammal, preferably from a mammal recipient.

13. A biological cell containing the modified *Parapoxvirus* or *Parapoxvirus* vector of any of the preceding claims, preferably a mammalian cell, further preferably a Vero cell, a HEK 293 cell or an antigen-presenting cell.

14. A pharmaceutical composition, preferably a vaccine, containing the modified *Parapoxvirus* or *Parapoxvirus* vector of any of claims 1-12 or/and the cell of claim 13, and a pharmaceutically acceptable carrier.

15. Use of a modified *Parapoxvirus* or *Parapoxvirus* vector comprising at least one functional mutation in the viral open reading frame (ORF) encoding the 'chemokine binding protein' (CBP) for the induction of an immune response in a living being, preferably a mammal or a human being.
